# EUROPEAN PATENT APPLICATION

(11) **EP 2 770 341 A1**
(43) Date of publication of application: **27.08.2014**
(21) Application number: 11874135.4
(22) Date of filing: 19.10.2011
(51) Int. Cl.: G01T 1/161, G01N 21/64, G01N 33/49

(54) **DISPLAY DEVICE, DISPLAY METHOD, AND DISPLAY PROGRAM, USED IN MEASUREMENT SYSTEM**

(71) Applicant: Shimadzu Corporation, Kyoto-shi, Kyoto 604-8511 (JP)
(72) Inventor: HASHIZUME, Nobuya, Kyoto-shi, Kyoto 604-8511 (JP); TSUDA, Tomoaki, Kyoto-shi, Kyoto 604-8511 (JP); AKECHI, Masakazu, Kyoto-shi, Kyoto 604-8511 (JP); OISHI, Akira, Kyoto-shi, Kyoto 604-8511 (JP)
(74) Representative: Webster, Jeremy Mark
(86) International application number: PCT/JP2011/005859
(87) International publication number: WO 2013/057762

(57) **Abstract**

The display apparatus of this invention, even when edges of an IP image with measurement information on β⁺ rays are indistinct and an area to be measured is unclear, includes a main screen 51 for displaying in superimposition the IP image and a scanner image with morphological information on blood, whereby an area measured of the scanner image can be grasped visually from the IP image. Even if traces of movement of the blood appear on the IP image, the position of an object to be measured can be determined by displaying the IP image and the scanner image in superimposition.

## Description

### Technical Field

This invention relates to a display apparatus, a display method and a display program for use in a measuring system for measuring light generated from a luminescent or fluorescent substance included in a liquid to be measured, or radiation included in the liquid to be measured.

### Background Art

The measuring system is used in a liquid collecting apparatus, for example. The liquid collecting apparatus will be described taking a blood collecting apparatus which collects blood, for example. The blood collecting apparatus is used for quantitative analysis in nuclear medicine diagnosis (e.g. PET (Positron Emission Tomography), SPECT (Single Photon Emission CT) and so on), and is used especially for measurement of a radioactive concentration in arterial blood of small animals (e.g. mice, rats and so on).

Specifically, blood is collected from a small animal medicated with a radioactive drug, plasma separation by centrifugal separation is carried out after the end of a whole blood collection for each predetermined time, and time variations of radioactive concentration in the whole blood and plasma are measured (see Patent Document 1, for example). To describe this more particularly, measurement is carried out using an imaging plate (IP) which visualizes radioactivity distributions by exposing β⁺ rays included in blood. As software for acquiring values of radiation dose from IP images obtained from the imaging plate (hereinafter abbreviated as "IP" as appropriate), there is a Multi Gauge made by Fujifilm Corporation, for example. With this software, a radiation dose per unit area can be obtained by reading an IP image, setting an area of interest on the software, and calculating pixel values in the area of interest.

### [Prior Art Document]

### [Patent Document]

[Patent Document 1]
International Publication No. WO2009-093306

### Summary of Invention

### Technical Problem

However, with the conventional technique, since an IP image has only a radiation distribution imaged as noted above, there is a problem that edges are indistinct and the position of an object measured is visually unclear. Where the object measured is a liquid including a radioactive substance, the radioactive substance adheres also to traces of movement of the liquid measured and appears on the IP image. There is a problem, therefore, that it is difficult to determine the position of the object measured only by means of the measurement information image represented by the IP image or the like. It is conceivable to use a method of using a morphological information image represented by a scanner image or the like in order to set an accurate area of interest. However, it has proved impossible, with conventional analysis software dedicated to IP images, including Multi Gauge, to read and analyze images other than those obtained with IP image readers.

This invention has been made having regard to the state of the art noted above, and its object is to provide a display apparatus, a display method and a display program for use in a measuring system, in which an area of an object to be measured can be grasped from a measurement information image.

### Solution to Problem

To solve the above problem, Inventors have made intensive research and attained the following findings.

Without using anything other than the conventional analysis software dedicated to IP images, by carrying out a superimposition process to superimpose, by a CPU (central processing unit), for example, a measurement information image represented by an IP image or the like and a morphological information image represented by a scanner image or the like, an area of an object to be measured of the measurement information image can be grasped from the morphological information image, which makes it possible to determine the position of the object to be measured. However, since the superimposition process is carried out according to an algorithm by the CPU, the superimposition process can be automatically carried out in error if there is a fault on the part of the morphological information image. Further, when a liquid which is the object to be measured moves, traces of the movement can be recognized in error to be the object of the superimposition process.

Then, a technique for performing calibration with regard to the superimposition process is also conceivable in which a superimposition process is carried out, based on density variations, by moving plasma and blood cell areas in a scanner image vertically and horizontally, and determining a position of maximum overlap with an IP image to be a position of superimposition. However, when the above-noted traces of movement are recognized in error as positions of superimposition based on the density variations, the calibration itself can be carried out automatically in error. Then, it has been found that areas of the object to be measured can be confirmed visually by returning to the starting line and visualizing and displaying these images, instead of relaying on an automatic analysis based on the algorithm by the CPU.

Based on such findings, this invention provides the following construction.

A display apparatus according to this invention is a display apparatus for use in a measuring system for measuring light generated from a luminescent or fluorescent substance included in a liquid to be measured, or radiation included in the liquid to be measured, the display apparatus comprising a first reading device for reading a measurement information image with measurement information on the light generated from the luminescent or fluorescent substance included in the liquid to be measured, or the radiation included in the liquid to be measured; a second reading device for reading a morphological information image with morphological information on the liquid to be measured; and a superimposition displaying device for displaying in superimposition the measurement information image read by the first reading device and the morphological information image read by the second reading device.

The display apparatus for use in the measuring system according to this invention, even when edges of the measurement information image with measurement information on light or radiation are indistinct and an area to be measured is unclear, includes the first reading device for reading the measurement information image thereof, the second reading device for reading the morphological information image with morphological information on the liquid to be measured, and the superimposition displaying device for displaying in superimposition the measurement information image read by the first reading device and the morphological information image read by the second reading device, whereby the area to be measured of the morphological information image can be grasped visually from the measurement information image. Even if traces of movement of the liquid measured appear on the measurement information image, the position of the object measured can be determined by the superimposition displaying device which displays the measurement information image and the morphological information image in superimposition.

A display method according to this invention is a display method for displaying measurement data obtained by measuring light generated from a luminescent or fluorescent substance included in a liquid to be measured, or radiation included in the liquid to be measured, the display method comprising a first reading step for reading a measurement information image having measurement information on the light generated from the luminescent or fluorescent substance included in the liquid to be measured, or the radiation included in the liquid to be measured; a second reading step for reading a morphological information image having morphological information on the liquid to be measured; and a superimposition displaying step for displaying in superimposition the measurement information image read in the first reading step and the morphological information image read in the second reading step.

A display program according to this invention is a display program for causing a computer to execute a series of processes for displaying measurement data obtained by measuring light generated from a luminescent or fluorescent substance included in a liquid to be measured, or radiation included in the liquid to be measured, the display program comprising a first reading step for reading a measurement information image having measurement information on the light generated from the luminescent or fluorescent substance included in the liquid to be measured, or the radiation included in the liquid to be measured; a second reading step for reading a morphological information image having morphological information on the liquid to be measured; and a superimposition displaying step for displaying in superimposition the measurement information image read in the first reading step and the morphological information image read in the second reading step; the computer being caused to carry out processes in the above steps.

According to the display method and display program of this invention, the first reading step reads the measurement information image with measurement information on the light generated from the luminescent or fluorescent substance included in the liquid to be measured, or the radiation included in the liquid to be measured, and the second reading step reads the morphological information image with morphological information on the liquid to be measured. And the measurement information image read in the first reading step and the morphological information image read in the second reading step are superimposed and displayed in the superimposition displaying step, whereby an area to be measured of the morphological information image can be grasped visually from the measurement information image.

In the display apparatus according to this invention described above, it is preferred to comprise an edge enhancing device for enhancing edges of the morphological information image read by the second reading device and outputting an edge-enhanced image; wherein the superimposition displaying device displays in superimposition the measurement information image read by the first reading device and the edge-enhanced image with the edges enhanced by the edge enhancing device. In the display method according to this invention described above, it is preferred to comprise an edge enhancing step for enhancing edges of the morphological information image read in the second reading step and outputting an edge-enhanced image; wherein the superimposition displaying step displays in superimposition the measurement information image read in the first reading step and the edge-enhanced image with the edges enhanced in the edge enhancing step. An area of interest can be extracted by enhancing the edges of the morphological information image and outputting the edge-enhanced image, and can easily be discerned at the time of superimposition display by superimposing it with the measurement information image.

The display apparatus described above may comprise a measurement information image displaying device for displaying the measurement information image read by the first reading device, and may comprise a morphological information image displaying device for displaying the morphological information image read by the second reading device. By displaying at least one of the measurement information image and the morphological information image serving as the basis of superimposition display, each image serving as the basis of superimposition display can be visually discerned with ease.

In the display apparatus described above, the respective images may be displayed on one screen. The images can be made visually further discernible by quickly switching the images for display on one screen. Of course, the respective images may be displayed on one window screen, and also the respective images may be displayed on one or more tabs or one or more window screens different from that one window screen.

In the display apparatus according to this invention described above, it is preferred to comprise a reduced displaying device for displaying in reduction at least one of the measurement information image read by the first reading device and the morphological information image read by the second reading device; an image selecting device for selecting the image displayed in reduction by the reduced displaying device; and an enlarged displaying device for displaying in enlargement the image selected by the image selecting device.

In the display method according to this invention described above, it is preferred to comprise a reduced displaying step for displaying in reduction at least one of the measurement information image read in the first reading step and the morphological information image read in the second reading step; an image selecting step for selecting the image displayed in reduction in the reduced displaying step; and an enlarged displaying step for displaying in enlargement the image selected in the image selecting step.

By displaying in reduction at least one of the measurement information image and the morphological information image serving as the basis of superimposition display, the image displayed in reduction can be selected with ease. Further, by displaying in enlargement the image selected, the image selected can be visually discerned with ease.

Where the reduced displaying device, the image selecting device and the enlarged displaying device are provided, it is preferred that the reduced displaying device displays in reduction an image after the superimposition process which superimposes the measurement information image and the morphological information image, the image selecting device is constructed capable of selecting also the image after the superimposition process displayed in reduction on the reduced displaying device, and the enlarged displaying device, when the image after the superimposition process is selected by the image selecting device, displays the image after the superimposition process in enlargement.

Where the reduced displaying step, the image selecting step and the enlarged displaying step are provided, it is preferred that the reduced displaying step displays in reduction an image after the superimposition process which superimposes the measurement information image and the morphological information image, the image selecting step selects the image after the superimposition process displayed in reduction in the reduced displaying step, and the enlarged displaying step, when the image after the superimposition process is selected in the image selecting step, displays the image after the superimposition process in enlargement.

By displaying in reduction the image after the superimposition process, the image after the superimposition process displayed in reduction can also be selected with ease. Further, by displaying in enlargement the image after the superimposition process selected, a switching display can be made with ease visually between the image after the superimposition process selected and each image that can be selected.

Also where the reduced displaying step, the image selecting step and the enlarged displaying step are provided, the respective images including the image displayed in reduction and the image displayed in enlargement may be displayed on one screen. The images can be made visually further discernible by quickly switching the images for display on one screen. Of course, the respective images including the image displayed in reduction and the image displayed in enlargement may be displayed on one window screen, and also the respective images may be displayed on one or more tabs or one or more window screens different from that one window screen.

### Advantageous Effects of Invention

The display apparatus for use in the measuring system according to this invention includes a first reading device for reading a measurement information image with measurement information on light generated from a luminescent or fluorescent substance included in a liquid to be measured, or radiation included in the liquid to be measured; a second reading device for reading a morphological information image with morphological information on the liquid to be measured; and a superimposition displaying device for displaying in superimposition the measurement information image read by the first reading device and the morphological information image read by the second reading device, whereby an area measured of the morphological information image can be grasped visually from the measurement information image.

According to the display method and display program of this invention, a first reading step reads the measurement information image with measurement information on the light generated from the luminescent or fluorescent substance included in the liquid to be measured, or the radiation included in the liquid to be measured, and a second reading step reads the morphological information image with morphological information on the liquid to be measured. And the measurement information image read in the first reading step and the morphological information image read in the second reading step are superimposed and displayed in a superimposition displaying step, whereby the area to be measured of the morphological information image can be grasped visually from the measurement information image.

### Brief Description of Drawings

Figure 1 is a schematic perspective view of a blood collecting apparatus and a measuring apparatus according to each embodiment;
Figure 2 is a block diagram of the measuring apparatus according to each embodiment;
Figure 3 is a schematic perspective view of a scanner in an image pickup unit of the measuring apparatus;
Figure 4 is a schematic plan view of a disk according to each embodiment;
Figure 5 is a block diagram of a display apparatus according to each embodiment;
Figure 6 shows one example of display modes on an output monitor of the display apparatus;
Figure 7 is a flow chart showing a sequence of a series of steps according to Embodiment 1; and
Figure 8 is a flow chart showing a sequence of a series of steps according to Embodiment 2.

### Embodiment 1

Embodiment 1 of this invention will be described hereinafter with reference to the drawings.

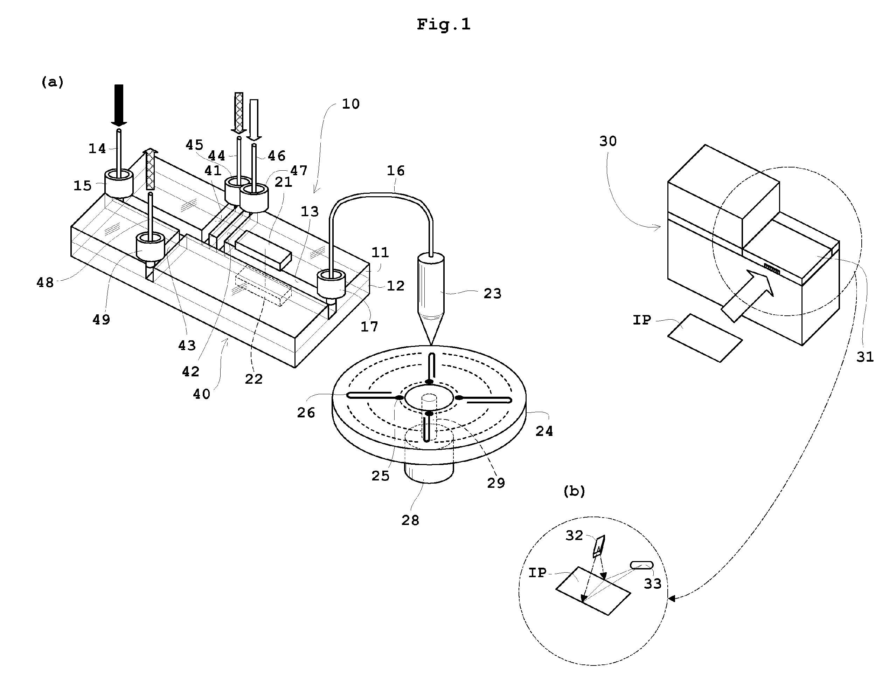

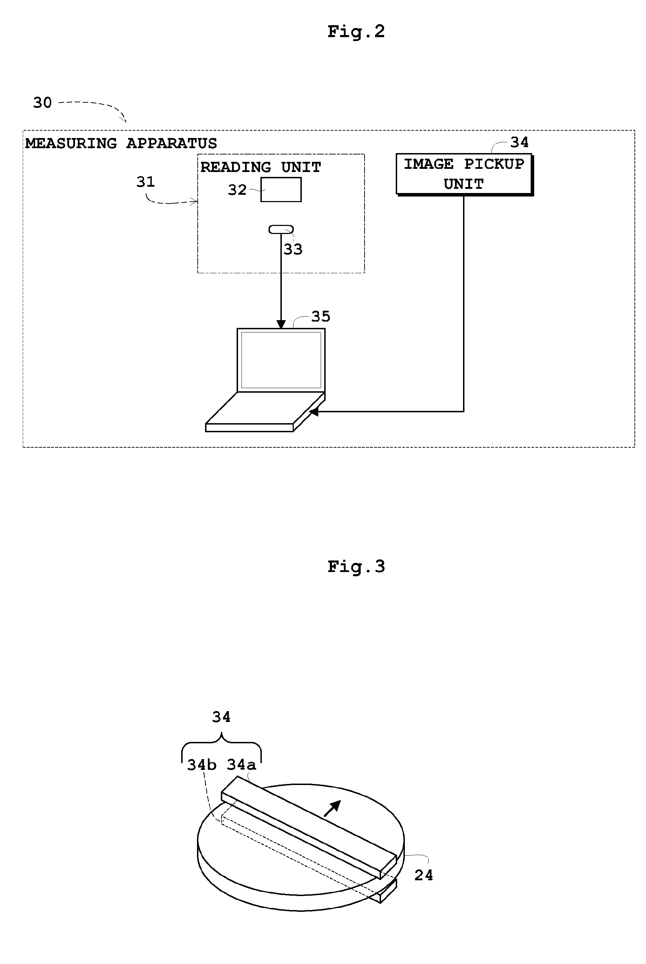

Fig. 1 is a schematic perspective view of a blood collecting apparatus and a measuring apparatus according to each embodiment. Fig. 2 is a block diagram of the measuring apparatus according to each embodiment. Fig. 3 is a schematic perspective view of a scanner in an image pickup unit of the measuring apparatus. This Embodiment 1, including Embodiment 2 to be described hereinafter, will be described, taking blood as an example of liquid to be measured, and taking a system having a blood collecting apparatus and a measuring apparatus as an example of measuring system.

As shown in Fig. 1, a blood collecting apparatus 10 according to this Embodiment 1, including Embodiment 2 to be described hereinafter, collects blood to be measured, as separated in a time series. Around the blood collecting apparatus 10, a measuring apparatus 30 is provided for measuring radiation (eg β-rays, γ-rays or the like) included in the blood collected by the blood collecting apparatus 10.

The blood collecting apparatus 10 has a microfluidic device (liquid dividing device) 40 formed of two PDMS substrates 11 and 12 of PDMS (Polydimethylsiloxane) resin, one superposed on the other. The PDMS substrates 11 and 12 are grooved in a predetermined size, and grooves resulting from the grooving form a main flow path 13 and side paths 41, 42 and 43. Here, the material for the blood collecting apparatus 10 is not limited to PDMS, but may be any resin optically transparent material such as acrylic, polycarbonate, COP (cycloolefin polymer) or the like.

A catheter 14 is disposed at a blood inlet of the main flow path 13, and the main flow path 13 and catheter 14 are connected through a connector 15. Blood is continuously fed from the catheter 14 into the main flow path 13, and its inflow volume is controlled by a valve (not shown). Blood piping 16 is disposed at a blood outlet of the main flow path 13, and the main flow path 13 and blood piping 16 are connected through a connector 17.

A light source 21 and a photodiode 22 are opposed to each other across the main flow path 13. Light is emitted from the light source 21 to the blood, or a heparin solution to be described hereinafter, flowing through the main flow path 13, and the photodiode 22 detects light-shielding by the blood, thereby to measure length information on the blood or heparin solution described hereinafter while optically monitoring the blood or heparin solution. Although the light source 21 and photodiode 22 have been described here as an example of the optical measuring device, the light source 21 and photodiode 22 are not limitative as long as the device measures the intervals of the liquid while optically monitoring the liquid to be measured. For example, volume information of the liquid to be measured may be acquired with a CCD camera. The light source 21 and photodiode 22 are opposed to each other across the main flow path 13 as shown in Fig. 1, to constitute what is called a "transmission type sensor" for making detection based on light-shielding by the blood. Instead, what is called a "reflection type sensor" may be used to detect light reflected by the blood, with a light detecting device represented by a photodiode arranged at the same side as the light source.

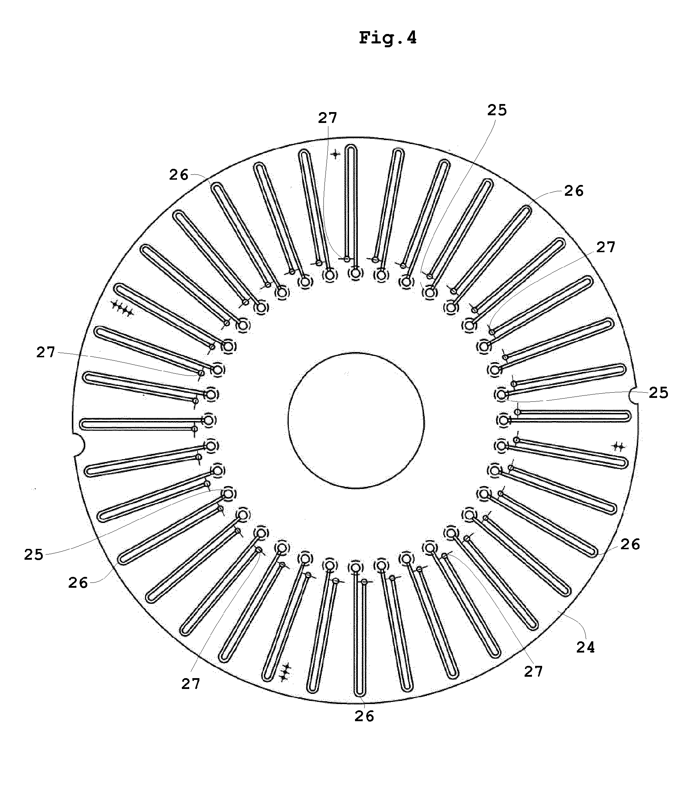

On the other hand, a nozzle 23 is connected downstream of the above blood piping 16. A capillary tube such as a hypodermic needle or glass tube is used as the nozzle 23. Although the nozzle 23 is used as a discharger to discharge the liquid here, a dispenser may be used instead. A disk (also called "CD well") 24 is disposed for receiving and storing the blood dripping from this nozzle 23. The disk 24 has a plurality of flow path inlets 25 in form of openings (see Fig. 4 also) arranged centrally and radially thereof for receiving the dripping blood. The disk 24 is grooved, as is the above PDMS substrates 11 and 12, and a plurality of U-shaped flow paths 26 in form of U-shaped grooves (see Fig. 4 also) are formed radially by the grooving. Each U-shaped flow path 26 is connected in a one-to-one relationship to one outer end of the above flow path inlet 25, and each U-shaped flow path 26 is formed to extend radially of the disk 24. With the nozzle 23 interposed in this way, the disk 24 is formed capable of circulating blood with respect to the main flow path 13. A specific construction of the disk 24 will be described hereinafter with reference to Fig. 4 et seq.

On the other hand, the measuring apparatus 30 has a reading unit 31. This reading unit 31 has a cover for inserting an exposed imaging plate IP, and detects β⁺ rays included in the blood by reading excited light from the imaging plate IP. Specifically, as shown in Fig. 1 (b), the reading unit 31 has a laser light source 32 and a photomultiplier tube 33. β⁺ rays are simultaneously detected in two dimensions by emitting laser from the laser light source 32 to the imaging plate IP, with the photomultiplier tube 33 converting to and multiplying electrons, the light excited by the laser emission to the imaging plate IP.

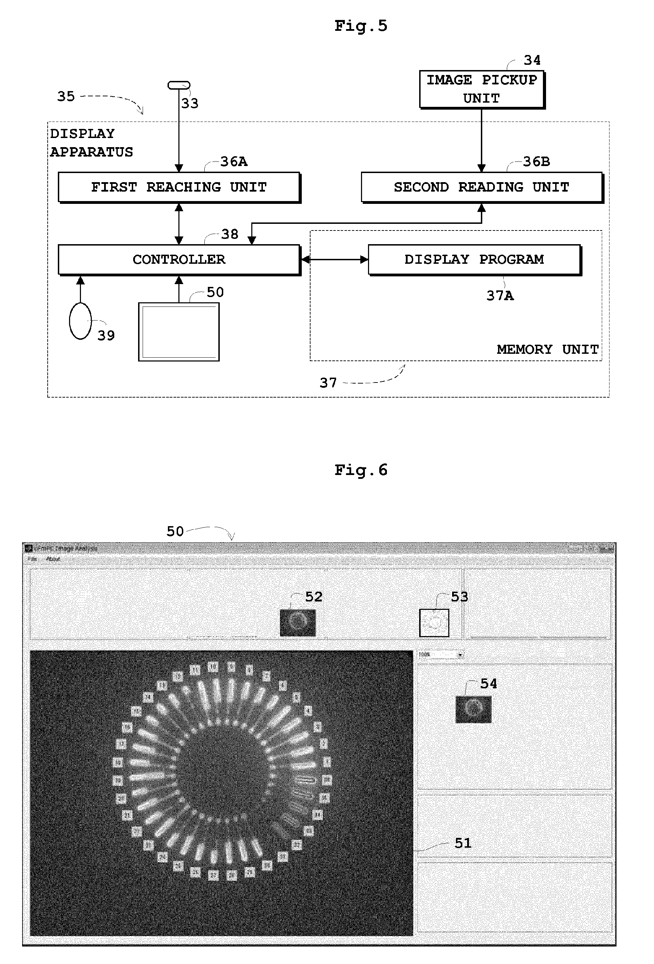

A block diagram of the measuring apparatus 30 will be described next. As shown in Fig. 2, the measuring apparatus 30 includes, besides the above reading unit 31, an image pickup unit 34 and a display apparatus 35. The display apparatus 35 may be formed of an ordinary personal computer. The display apparatus 35 corresponds to the display apparatus in this invention. A specific construction of the display apparatus 35 will be described hereinafter with reference to Fig. 5 et seq.

As shown in Fig. 3, the image pickup unit 34 picks up an image of the disk 24. In this Embodiment 1, including Embodiment 2 to be described hereinafter, a flathead scanner is employed as the image pickup unit 34. The flathead scanner includes a linear light source 34a having a length at least corresponding to the diameter of the disk 24, and a linear photodiode array (i.e. a line sensor) 34b opposed to the light source 34a across the disk 24. The flathead scanner scans over the disk 24 to pick up an image of the disk 24, thereby acquiring the image of the disk 24.

Returning to the description of Fig. 1, the microfluidic device 40 has the main flow path 13 for feeding blood, a side path 41 for feeding the heparin solution which is a type of anticoagulant for preventing an occurrence of blood coagulation, a side path 42 for feeding air or gas, and a side path 43 for discharging the blood or heparin solution.

Cleaning solution piping 44 is disposed at a solution inlet of the side path 41, and the side path 41 and cleaning solution piping 44 are connected through a connector 45. The flow paths are cleaned as necessary by pouring the heparin solution from the cleaning solution piping 44 through the side path 41 into the main flow path 13. Inflow volume of the heparin solution is controlled by a valve. The anticoagulant is not limited to the heparin solution.

Bubble piping 46 is disposed at a gas inlet of the side path 42, and the side path 42 and bubble piping 46 are connected through a connector 47. Air or gas controlled by a pressure generator (not shown) is fed through the side path 42 into the main flow path 13, with its feed time adjusted by a valve. With these bubbles, blood is extracted based on length information on the blood, and waste liquids (blood, heparin solution or mixture of these) remaining in the flow paths of the microfluidic device 40 are discharged. There is no limitation regarding the gas to be fed, but may be any gas that does not react with blood or heparin solution, which may be a rare gas such as helium, neon or argon, or nitrogen gas, for example.

The bubble piping 46 feeds the gas (e.g. air or gas) into the main flow path 13 through the side path 14, introducing the gas as bubbles at specified predetermined intervals, thereby feeding the blood to be measured, as separated in a time series, to the disk 24. That is, the bubbles perform a function as separators. Although a gas is used as the separators, instead of being limited to the gas, a liquid other than the liquid to be measured may be used as the separators as long as this liquid has little or no chance of mixing with the liquid to be measured (blood in Embodiments 1 and 2). Where the liquid to be measured is blood as in this Embodiment 1, including Embodiment 2 to be described hereinafter, a liquid represented by mineral oil, fluorine-based oil or the like, which does not mix with blood, may be used as the separators. However, using a liquid as the separators, although this is possible, is not desirable in terms of feeding to and collecting from the disk 24 because of contact with blood.

Waste liquid piping 48 is disposed at a waste liquid outlet of the side path 43, and the side path 43 and waste liquid piping 48 are connected through a connector 49. An amount of discharge is adjusted with a valve, to discharge as waste liquid part of blood other than the blood to be collected, the heparin solution after flow path cleaning, and a mixture of these.

A valve is disposed downstream of the connector 15 of the main flow path 13. A valve is disposed upstream of the connector 17, light source 21 and photodiode 22 of the main flow path 13. A valve is disposed downstream of the connector 45 of the side path 41. A valve is disposed downstream of the connector 47 of the side path 42. A valve is disposed upstream of the connector 49 of the side path 43.

Next, a specific construction of the disk 24 will be described with reference to Fig. 4 as well as Fig. 1. Fig. 4 is a schematic plan view of the disk according to each embodiment. As shown in Fig. 4, the U-shaped flow paths 26 of the disk 24 are formed to connect the flow path inlets 25 described hereinbefore and air holes 27. The U-shaped flow paths 26 are U-shaped so that, when the flow path inlets 26 acting as blood feed ports are regarded as being located upstream, and the air holes 27 as being located downstream, each flow path 26 extends from upstream to downstream, i.e. from a radially inward position to a radially outward position of the disk 24, and then turns back to extend radially inward from the radially outward position of the disk 24. A plurality of such flow paths 26 are provided.

As shown in Fig. 1, a motor 28 is provided centrally of the disk 24 for rotating the disk 24. The motor 28 has a rotary shaft 29 connected to the disk 24, whereby the centrifugal force of the disk 24 generated by the motor 28 is used to carry out plasma separation to centrifuge the blood into plasma and blood cell.

In this Embodiment 1, including Embodiment 2 to be described hereinafter, the disk 24 is formed of an acrylic plate. The material for the disk 24 is not limited to acrylic, but may be PDMS noted hereinbefore, or any other resin optically transparent material such as polycarbonate, COP or the like.

Next, a specific construction of the display apparatus 35 will be described with reference to Figs. 5 and 6. Fig. 5 is a block diagram of the display apparatus according to each embodiment. Fig. 6 shows one example of display modes on an output monitor of the display apparatus. As shown in Fig. 5, the display apparatus 35 includes a first reading unit 36A, a second reading unit 36B, a memory unit 37, a controller 38, an input unit 39 and an output monitor 50. The first reading unit 36A corresponds to the first reading device in this invention. The second reading unit 36B corresponds to the second reading device in this invention. The controller 38 corresponds to the edge enhancing device in this invention. The input unit 39 corresponds to the image selecting device in this invention.

The first reading unit 36A and second reading unit 36B are provided by a reading device such as an I/O (Input/Output) device, for example. The first reading unit 36A reads an IP image acquired by the imaging plate IP (see Fig. 1) through the reading unit 31 (see Figs. 1 and 2). The second reading unit 36B reads a scanner image acquired by the image pickup unit 34. The IP image corresponds to the measurement information image in this invention. The scanner image corresponds to the morphological information image in this invention.

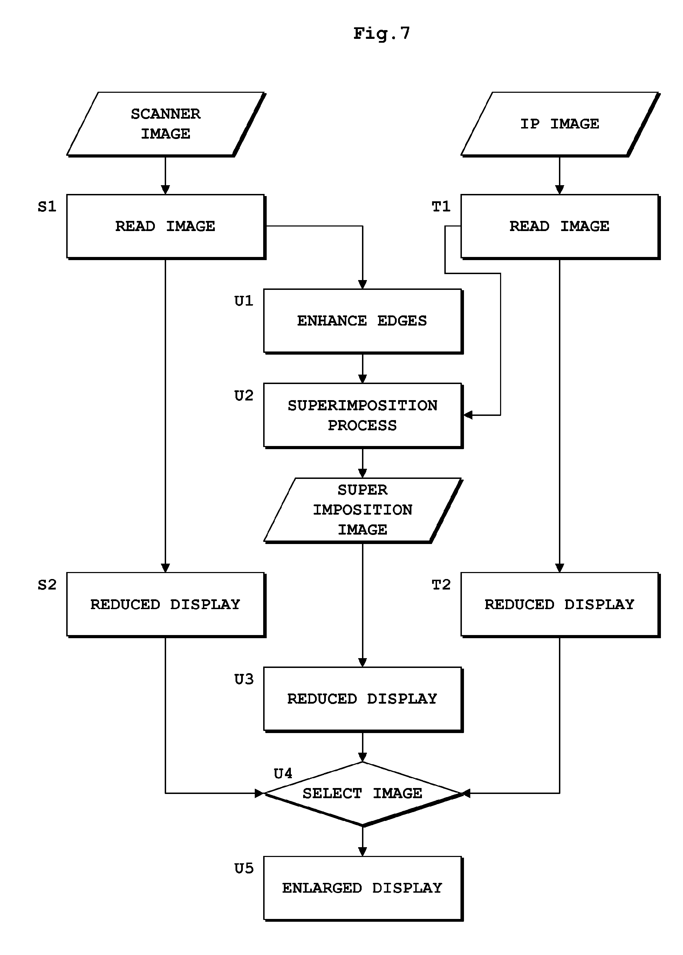

The memory unit 37 is in form of a storage medium represented by a ROM (Read-only Memory), RAM (Random Access Memory) or the like. In this Embodiment 1, including Embodiment 2 to be described hereinafter, the memory unit 37 has a display program 37A for causing a computer (controller 38 in each embodiment) to execute a series of steps shown in Fig. 7. The display program 37A is provided by a ROM. The display program 37A corresponds to the display program in this invention.

The controller 38 is in form of a central processing unit (CPU), for example. The controller 38 executes programs for carrying out various image processing, a program for calculating radioactive concentrations, and the display program 37A shown in Fig. 5, to perform image processes corresponding to these programs and the series of steps (reading controls for the first/second reading units 36A and 36B and display mode on the output monitor 50) shown in Fig. 7 and corresponding to the display program 37A. As the various image processes, the controller 38 has functions for reduction and enlargement processes to reduce or enlarge the scanner image, and functions of the edge enhancing device for enhancing edges of the scanner image and outputting an edge-enhanced image.

The input unit 39 is in form of a pointing device represented by a mouse, keyboard, joystick, trackball or touch panel. In this Embodiment 1, including Embodiment 2 to be described hereinafter, the input unit 39 has functions of the image selecting device to select each image displayed in reduction on reduced screens 52, 53 and 54 (see Fig. 6) described hereinafter, in order to display it in enlargement on a main screen 51 (see Fig. 6) described hereinafter.

The output monitor 50, as shown in Fig. 6, includes a main screen 51 for displaying a main image in enlargement, a reduced screen 52 for displaying an IP image in reduction, a reduced screen 53 for displaying a scanner image in reduction, and a reduced screen 54 for displaying in reduction an image resulting from an superimposition process which superimposes the IP image and scanner image. In Fig. 6, an IP image read by the first reading unit 36A and a scanner image read by the second reading unit 36B are displayed in superimposition on the main screen 51. The main screen 51 corresponds to the superimposition displaying device and the enlargement displaying device in this invention. The reduced screen 52 (for displaying an IP image in reduction) corresponds to the measurement information image displaying device and the reduced displaying device in this invention. The reduced screen 53 (for displaying a scanner image in reduction) corresponds to the morphological information image displaying device and the reduced displaying device in this invention. The reduced screen 54 (for displaying in reduction an image resulting from a superimposition process) corresponds to the reduced displaying device in this invention.

Although, in Fig. 6, the reduced displaying devices are divided into the reduced screens 52, 53 and 54 for the respective images, they may be combined into one or two switchable to give displays in reduction of selected images. In Fig. 6, all the images displayed in reduction have the same size, and the images displayed in enlargement have the same size. Of course, the images displayed in reduction may be different in size from one another, and the images displayed in enlargement may be different in size from one another.

The centrifugal force of the disk 24 (see Figs. 1, 3 and 4) generated by the motor 28 (see Fig. 1) effects plasma separation into plasma and blood cell, and the disk 24 is imaged in this state. An IP image is acquired by imaging the disk 24 using the imaging plate IP (see Fig. 1), and a scanner image is acquired by imaging the disk 24 with the flathead scanner of the image pickup unit 34 (see Figs. 2, 3 and 5). There is no limitation to a particular order of image pickup.

To describe this specifically, each disk 24 (see Figs. 1, 3 and 4) with the plasma and blood cell having undergone the plasma separation is, as a sample, put into an open cassette not shown, an imaging plate IP (see Fig. 1) is placed on the sample, and the cassette is closed for exposure. Through this exposure, electrons are captured by lattice defects of a phosphor (not shown) of the imaging plate IP with the ionization power of β⁺ rays included in the blood. After a fixed time of exposure, the imaging plate IP is taken out of the cassette, and is inserted into the cover of the reading unit 31 (see Figs. 1 and 2) of the measuring apparatus 30 (see Fig. 1), and the imaging plate IP is exposed to light emitted thereto.

The laser light source 32 (see Figs. 1 and 2) of the reading unit 31 (see Figs. 1 and 2) emits laser to the imaging plate IP (see Fig. 1). The captured electrons are excited by this irradiation to conductors to recombine with holes, which are excited as light from the phosphor. The photomultiplier tube 33 (see Figs. 1, 2 and 5) converts the light excited by the laser irradiation of the imaging plate IP into electrons and multiplies the electrons, thereby simultaneously detecting the electrons in two dimensions and counting them as electric pulses. After laser is emitted from the laser light source 32 to the imaging plate IP, the captured electrons are erased by light emitted from an erasing light source (not shown) to the imaging plate IP for reuse. Based on count information on β⁺ rays obtained from the imaging plate IP and reading unit 31, a dose of radiation in the blood which is count information on β⁺rays is obtained. An IP image is acquired in this way.

On the other hand, the image pickup unit 34 (see Figs. 2, 3 and 5) picks up an image of the plasma and blood cell resulting from the plasma separation for each disk 24 (see Figs. 1, 3 and 4). By emitting light from the light source 32a (see Fig. 3) of the flathead scanner of the image pickup unit 34, differences in absorbance cause the plasma and blood cell to appear as shading differences on the image picked up, which can be distinguished easily on the image. A scanner image is acquired in this way.

Next, a series of steps will be described with reference to Fig. 7. Fig. 7 is a flow chart showing a sequence of a series of steps according to Embodiment 1.

First, through the reading unit 31 (see Figs. 1 and 2), an IP image is acquired from the imaging plate IP (Fig. 1), and a scanner image is acquired from the flathead scanner of the image pickup unit 34 (see Figs. 2, 3 and 5).

### (Step S1) Read image

The second reading unit 36B (see Fig. 5) reads the scanner image acquired by the image pickup unit 34 (see Figs. 2, 3 and 5). This step S1 corresponds to the second reading step in this invention.

### (Step U1) Edge enhancement

In order to extract an area of interest, the controller 38 (see Fig. 5) enhances edges of the scanner image read in step S1, and outputs an edge-enhanced image. An edge enhancing process may consist of an edge enhancing process by primary differentiation which determines differences between an attention pixel and its peripheral pixels, and which is represented, for example, by Sobel filter process or Prewitt filter process, and an edge enhancing process by secondary differentiation which determines further differences of the differences between the attention pixel and its peripheral pixels, and which is represented, for example, by Laplacian filter process. These edge enhancing processes are known techniques and therefore their description is omitted. This step U1 corresponds to the edge enhancing step in this invention.

### (Step T1) Read image

On the other hand, the first reading unit 36A (see Fig. 5) reads the IP image acquired from the imaging plate IP (see Fig. 1) through the reading unit 31 (see Figs. 1 and 2). There is no limitation to the order of the foregoing step S1 and this step T1. Step T1 may be executed first, step S1 may be executed first, or steps S1 and T1 may be executed simultaneously in parallel. This step TS1 corresponds to the first reading step in this invention.

### (Step U2) Superimposition process

A superimposition process is carried out to superimpose the IP image read in step T1 and the scanner image read in step S1. In this Embodiment 1, preferably, the superimposition process is carried out to superimpose the IP image read in step T1 and the edge-enhanced image with edges enhanced in step U1.

### (Step S2) Reduced display

The controller 38 (see Fig. 5) carries out a reduction process of the scanner image read in step S1, and displays a scanner image after the reduction process on the reduced screen 53 shown in Fig. 6. By this reduction process, the scanner image is displayed in reduction on the reduced screen 53. Instead of the scanner image with the edges not enhanced, the edge-enhanced image with the edges enhanced in step U1 may be displayed in reduction on the reduced screen 53. However, since this reduction display is a display process for image selection in step U4 and is not a detailed display, it is not absolutely necessary to display, in reduction on the reduced screen 53, the edge-enhanced image with the edges enhanced in step U1.

### (Step T2) Reduced display

On the other hand, the controller 38 (see Fig. 5) carries out a reduction process of also the IP image read in step T1, and displays an IP image after the reduction process on the reduced screen 52 shown in Fig. 6. By this reduction process, the IP image is displayed in reduction on the reduced screen 52.

### (Step U3) Reduced display

The controller 38 (see Fig. 5) carries out a reduction process of an image (indicated "SUPERIMPOSITION IMAGE" in Fig. 7) after the superimposition process having undergone the superimposition process in step U2, and displays the image after the superimposition process and after the reduction process on the reduced screen 54 shown in Fig. 6. By this reduction process, the image after the superimposition process is displayed in reduction on the reduced screen 54. This step U3, including the foregoing steps S2 and T2 also, corresponds to the reduced displaying step.

### (Step U4) Select image

In order to give an enlarged display in step U5, the input unit 39 (see Fig. 5) is operated to select an image from among the images (including the image after the superimposition process) displayed in reduction in steps S2, T2 and U3. From the output monitor 50 shown in Fig. 6, an image to be displayed in enlargement is selected by setting and clicking the pointer on the image to be displayed in enlargement. The image selection is not limited to the technique of setting and clicking the pointer. An image to be displayed in enlargement may be selected by setting and double-clicking the pointer on the image to be displayed in enlargement. The input unit 39 may be formed of a touch panel of the output monitor 50, and an image to be displayed in enlargement may be selected by directly touching, with a finger, the image to be displayed in enlargement on the touch panel of the output monitor 50. A "selection decision" key may be displayed on the output monitor 50, for example, and an image to be displayed in enlargement may be selected by clicking the "selection decision" key with the mouse or keyboard of the input unit 39. This step U4 corresponds to the image selecting step in this invention.

### (Step U5) Enlarged display

The controller 38 (see Fig. 5) carries out an enlargement process of the image selected in step U4, and displays the image after the enlargement process on the main screen 51 shown in Fig. 6. By this enlargement process, the image selected in step U4 is displayed in enlargement on the main screen 51. When the image after the superimposition process is selected in step U4, the image after the superimposition process is displayed in enlargement. In this case, this step U5 will also carry out the superimposition displaying step for displaying the IP image and the scanner image in superimposition. Therefore, this step U5 corresponds to the enlarged displaying device in this invention, and also corresponds to the superimposition displaying step in this invention.

The display apparatus 35 used in the measuring system according to this Embodiment 1, even when edges of the measurement information image (IP image in each embodiment) with measurement information on light or radiation (β⁺ rays in each embodiment) are indistinct and an area to be measured is unclear, includes the first reading unit 36A for reading the measurement information image (IP image) thereof, the second reading unit 36B for reading the morphological information image (scanner image in each embodiment) with morphological information on the liquid to be measured (blood in each embodiment), and the superimposition displaying device (main screen 51 in each embodiment) for displaying in superimposition the measurement information image (IP image) read by the first reading unit 36A and the morphological information image (scanner image) read by the second reading unit 36B, whereby the area to be measured of the morphological information image (scanner image) can be grasped visually from the measurement information image (IP image). Even if traces of movement of the liquid measured (blood) appear on the measurement information image (IP image), the position of the object measured can be determined by the superimposition displaying device (main screen 51) which displays the measurement information image (IP image) and the morphological information image (scanner image) in superimposition.

According to the display method and display program 37A in this Embodiment 1, step T1 is executed to read the measurement information image (IP image in each embodiment) with measurement information on radiation (β⁺ rays in each embodiment) included in the liquid to be measured (blood in each embodiment), and step S1 is executed to read the morphological information image (scanner image in each embodiment) with morphological information on the liquid to be measured (blood). And the measurement information image (IP image) read in step T1 and the morphological information image (scanner image) read in step S1 are superimposed and displayed in step U5, whereby the area to be measured of the morphological information image (scanner image) can be grasped visually from the measurement information image (IP image).

It is preferred that the display apparatus 35 according to this Embodiment 1 includes an edge enhancing device (controller 38 in each embodiment) for enhancing the edges of the morphological information image (scanner image in each embodiment) read by the second reading unit 36B and outputting an edge-enhanced image, wherein the superimposition displaying device (main screen 51 in each embodiment) displays in superimposition the measurement information image (IP image in each embodiment) read by the first reading unit 36A and the edge-enhanced image with the edges enhanced by the edge enhancing device (controller 38).

It is preferred that the display method in this Embodiment 1 includes step U1 of enhancing the edges of the morphological information image (scanner image in each embodiment) read in step S1 and outputting an edge-enhanced image, wherein step U5 displays in superimposition the measurement information image (IP image in each embodiment) read in step T1 and the edge-enhanced image with the edges enhanced in step U1. An area of interest can be extracted by enhancing the edges of the morphological information image (scanner image) and outputting the edge-enhanced image, and can easily be discerned at the time of superimposition display by superimposing it with the measurement information image (IP image).

The display apparatus 35 according to this Embodiment 1 includes a measurement information image displaying device (reduced screen 52 in each embodiment) for displaying the measurement information image (IP image in each embodiment) read by the first reading unit 36A, and includes a morphological information image displaying device (reduced screen 53 in each embodiment) for displaying the morphological information image (scanner image in each embodiment) read by the second reading unit 36B. By displaying at least one (both images: IP image and scanner image in each embodiment) of the measurement information image (IP image) and the morphological information image (scanner image) serving as the basis of superimposition display, each image serving as the basis of superimposition display can be visually discerned with ease.

It is preferred that the display apparatus 35 according to this Embodiment 1 includes a reduced displaying device (reduced screens 52, 53 and 54 in each embodiment) for displaying in reduction at least one (both images: IP image and scanner image in each embodiment) of the measurement information image (IP image in each embodiment) read by the first reading unit 36A and the morphological information image (scanner image in each embodiment) read by the second reading unit 36B, an image selecting device (input unit 39 in each embodiment) for selecting the image displayed in reduction by the reduced displaying device (reduced screens 52, 53 and 54), and an enlarged displaying device (main screen 51 in each embodiment) for displaying in enlargement the image selected by the image selecting device (input unit 39).

It is preferred that the display method in this Embodiment 1 includes steps S2, T2 and step U3 for displaying in reduction at least one of the measurement information image (IP image in each embodiment) read in step T1 and the morphological information image (scanner image in each embodiment) read in step S1, step U4 for selecting the image displayed in reduction in steps S2, T2 and step U3, and step U5 for displaying in enlargement the image selected in step U4.

By displaying in reduction at least one (both images: IP image and scanner image in each embodiment) of the measurement information image (IP image in each embodiment) and the morphological information image (scanner image in each embodiment) serving as the basis of superimposition display, the image displayed in reduction can be selected with ease. Further, by displaying in enlargement the image selected, the image selected can be visually discerned with ease.

Where the reduced displaying device (reduced screens 52, 53 and 54 in each embodiment), the image selecting device (input unit 39 in each embodiment) and the enlarged displaying device (main screen 51 in each embodiment) are provided as in this Embodiment 1, it is preferred that the reduced screen 54 displays in reduction an image after the superimposition process which superimposes the measurement information image (IP image in each embodiment) and the morphological information image (scanner image in each embodiment), the image selecting device (input unit 39) is constructed capable of selecting also the image after the superimposition process displayed in reduction on the reduced screen 54, and the main screen 51, when the image after the superimposition process is selected by the image selecting device (input unit 39), displays the image after the superimposition process in enlargement.

Where steps S2, T2 and step U3, step U4 and step U5 are provided as in this Embodiment 1, it is preferred that step U3 displays in reduction an image after the superimposition process which superimposes the measurement information image (IP image in each embodiment) and the morphological information image (scanner image in each embodiment), step U4 selects the image after the superimposition process displayed in reduction in the reduced displaying step, and step U5, when the image after the superimposition process is selected in step U4, displays the image after the superimposition process in enlargement.

By displaying in reduction the image after the superimposition process, the image after the superimposition process displayed in reduction can also be selected with ease. Further, by displaying in enlargement the image after the superimposition process selected, a switching display can be made with ease visually between the image after the superimposition process selected and each image that can be selected.

In this Embodiment 1, as shown in Fig. 6, the display apparatus 35 is constructed to display the respective images including the reduced images and the enlarged image on one screen (output monitor 50). The images can be made visually further discernible by quickly switching the images for display on the one screen.

### Embodiment 2

Next, Embodiment 2 of this invention will be described with reference to the drawings.

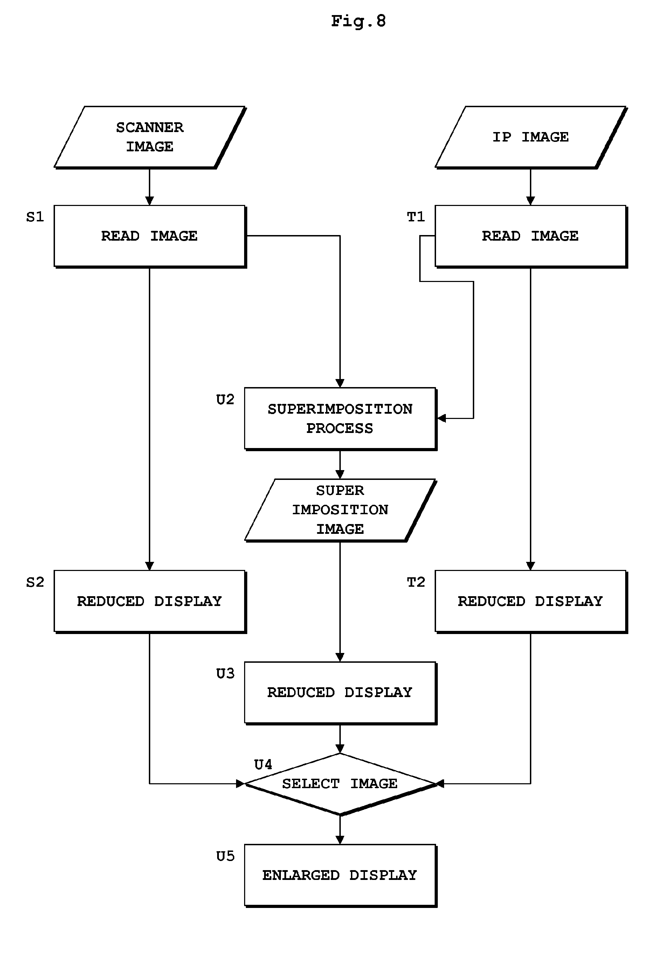

Fig. 8 is a flow chart showing a sequence of a series of steps according to Embodiment 2. The components common to foregoing Embodiment 1 will be affixed with the same signs, with their description omitted and illustration also omitted. The blood collecting apparatus and measuring apparatus according to this Embodiment 2 have the same constructions as in Figs. 1 - 3 described in Embodiment 1, and the disk and display apparatus according to this Embodiment 2 also have the same constructions as in Figs. 4 - 6 described in Embodiment 1. Thus, their description and illustration will be omitted.

The point which is different from the flow chart of Fig. 7 in Embodiment 1 lies in that the edge enhancement in step U1 shown in Fig. 7 is not done. That is, in Fig. 8, without carrying out the edge enhancement which enhances the edges in the scanner image read in step S1 and outputs the edge-enhanced image, the superimposition process is carried out (step U2) to superimpose the scanner image read in step S1 and the IP image read in step T1. The other steps are the same as the steps in the flow chart of Fig. 7 in Embodiment 1, and their description is omitted.

The functions and effects of this Embodiment 2 are the same as the functions and effects of Embodiment 1 except that the edge enhancement in Embodiment 1 is not carried out in this Embodiment 2, and their description is omitted.

This invention is not limited to the foregoing embodiments, but may be modified as follows:
(1) Each of the foregoing embodiments has been described taking blood as an example of the liquid to be measured. The liquid to be measured is not limited to blood, but may be a liquid including a radioactive substance, a luminescent substance or a fluorescent agent, or a mixed liquid used in an analyzing apparatus, for example.
   The liquid to be measured may be a liquid that is not subjected to centrifugal separation.
(2) In each of the foregoing embodiments, the measurement information image has been an IP image acquired from the imaging plate IP. However, it is not necessarily limited to the IP image, but may be a measurement information image with measurement information on light generated from a luminescent or fluorescent substance included in the liquid to be measured or on radiation included in the liquid to be measured, such as a measurement information image acquired by directly counting light (photons) or radiation, for example.
(3) In each of the foregoing embodiments, the morphological information image has been a scanner image acquired from the flathead scanner of the image pickup unit 34. However, it is not necessarily limited to the scanner image, but may be a morphological information image with morphological information on the liquid to be measured, such as a morphological information image acquired with a radiation image pickup device formed of a radiation emitting device and a radiation detecting device, for example.
(4) Each of the foregoing embodiments includes the measurement information image displaying device (reduced screen 52 in each embodiment) and the morphological information image displaying device (reduced screen 53 in each embodiment). However, it is not absolutely necessary to include the measurement information image displaying device (reduced screen 52) or the morphological information image displaying device (reduced screen 53) as long as the superimposition displaying device (main screen 51 in each embodiment) is provided as minimum requirement for finally giving a superimposed display.
(5) Each of the foregoing embodiments includes the reduced displaying device (reduced screens 52, 53 and 54 in each embodiment) for displaying in reduction at least one of the measurement information image (IP image in each embodiment) and the morphological information image (scanner image in each embodiment), but it is not absolutely necessary to include the reduced displaying device (reduced screens 52, 53 and 54). The reduced displaying device (reduced screens 52, 53 and 54) may display only the measurement information image (IP image) in reduction, or may display only the morphological information image (scanner image) in reduction. Reduced displays of the measurement information image (IP image) and the morphological information image (scanner image) may be combined into one on the same screen, and they may be switched for reduced display. Also when the image after the superimposition process is displayed in reduction, reduced displays of the measurement information image (IP image), the morphological information image (scanner image) and the image after the superimposition process may be combined into one or two on the same screen, and they may be switched for reduced display.
(6) In each of the foregoing embodiments, where the reduced displaying device (reduced screens 52, 53 and 54 in each embodiment) is provided, the input unit 39 acts as the image selecting device for selecting the images displayed in reduction by the reduced displaying device (reduced screens 52, 53, and 54) and the images are selected manually. However, the controller 38 may act the image selecting device to select the images automatically.
(7) In each of the foregoing embodiments, where the reduced displaying device (reduced screens 52, 53 and 54 in each embodiment) and the image selecting device (input unit 39 in each embodiment) are provided, the enlarged displaying device (main screen 51 in each embodiment) is provided for displaying in enlargement the image selected by the image selecting device (input unit 39). However, the enlarged displaying device (main screen 51) is not absolutely necessary. Further, the superimposition displaying device and the enlarged displaying device are combined into one screen (main screen 51 in each embodiment), but the superimposition displaying device and the enlarged displaying device may be separated to give displays, respectively.
(8) In each of the foregoing embodiments, where the reduced displaying device (reduced screens 52, 53 and 54 in each embodiment), the image selecting device (input unit 39 in each embodiment) and the enlarged displaying device (main screen 51 in each embodiment) are provided, the reduced displaying device (reduced screen 54) displays in reduction the image after the superimposition process, the image selecting device (input unit 39) is constructed capable of selecting also the image after the superimposition process which is displayed in reduction by the reduced displaying device (reduced screen 54), and the enlarged displaying device (main screen 51), when the image after the superimposition process is selected by the image selecting device (input unit 39), displays the image after the superimposition process in enlargement. However, it is not absolutely necessary to display also the image after the superimposition process in reduction or enlargement.
(9) Each of the foregoing embodiments provides a construction for displaying the respective images on one screen (output monitor 50 in each embodiment). As an alternative construction, the images may be displayed on one window screen, and also the images may be displayed on one or more tabs or one or more window screens different from that one window screen.
(10) Each of the foregoing embodiments includes the reduced displaying device (reduced screens 52, 53 and 54 in each embodiment) for displaying in reduction at least one of the measurement information image (IP image in each embodiment) and the morphological information image (scanner image in each embodiment), and further is constructed to display on one screen (output monitor 50) the respective images including the images displayed in reduction and the image displayed in enlargement. Instead, the respective images may be displayed on one screen (output monitor 50) without displaying them in reduction or enlargement. Similarly, without displaying them in reduction or enlargement, the respective images may be displayed on one or more tabs or one or more window screens different from that window screen.

### Reference Signs List

- 35: display apparatus
- 36A: first reading unit
- 36B: second reading unit
- 37A: display program
- 38: controller
- 39: input unit
- 51: main screen
- 52, 53, 54: reduced screens

## Claims

1. A display apparatus for use in a measuring system for measuring light generated from a luminescent or fluorescent substance included in a liquid to be measured, or radiation included in the liquid to be measured, the display apparatus comprising:
a first reading device for reading a measurement information image with measurement information on the light generated from the luminescent or fluorescent substance included in the liquid to be measured, or the radiation included in the liquid to be measured;
a second reading device for reading a morphological information image with morphological information on the liquid to be measured; and
a superimposition displaying device for displaying in superimposition the measurement information image read by the first reading device and the morphological information image read by the second reading device.

2. The display apparatus according to claim 1, comprising:
an edge enhancing device for enhancing edges of the morphological information image read by the second reading device and outputting an edge-enhanced image;
wherein the superimposition displaying device displays in superimposition the measurement information image read by the first reading device and the edge-enhanced image with the edges enhanced by the edge enhancing device.

3. The display apparatus according to claim 1 or 2, comprising a measurement information image displaying device for displaying the measurement information image read by the first reading device.

4. The display apparatus according to any one of claims 1 to 3, comprising a morphological information image displaying device for displaying the morphological information image read by the second reading device.

5. The display apparatus according to any one of claims 1 to 4, wherein the respective images are displayed on one screen.

6. The display apparatus according to any one of claims 1 to 4, wherein the respective images are displayed on one window screen, and also the respective images are displayed on one or more tabs or one or more different window screens different from that one window screen.

7. The display apparatus according to any one of claims 1 to 6, comprising:
a reduced displaying device for displaying in reduction at least one of the measurement information image read by the first reading device and the morphological information image read by the second reading device;
an image selecting device for selecting the image displayed in reduction by the reduced displaying device; and
an enlarged displaying device for displaying in enlargement the image selected by the image selecting device.

8. The display apparatus according to claim 7, wherein:
the reduced displaying device displays in reduction an image after a superimposition process which superimposes the measurement information image and the morphological information image;
the image selecting device is constructed capable of selecting also the image after the superimposition process displayed in reduction on the reduced displaying device; and
the enlarged displaying device, when the image after the superimposition process is selected by the image selecting device, displays the image after the superimposition process in enlargement.

9. The display apparatus according to claim 7 or 8, wherein the respective images including the image displayed in reduction and the image displayed in enlargement are displayed on one screen.

10. The display apparatus according to claim 7 or 8, wherein the respective images including the image displayed in reduction and the image displayed in enlargement are displayed on one window screen, and also the respective images are displayed on one or more tabs or one or more window screens different from that one window screen.

11. A display method for displaying measurement data obtained by measuring light generated from a luminescent or fluorescent substance included in a liquid to be measured, or radiation included in the liquid to be measured, the display method comprising:
a first reading step for reading a measurement information image having measurement information on the light generated from the luminescent or fluorescent substance included in the liquid to be measured, or the radiation included in the liquid to be measured;
a second reading step for reading a morphological information image having morphological information on the liquid to be measured; and
a superimposition displaying step for displaying in superimposition the measurement information image read in the first reading step and the morphological information image read in the second reading step.

12. The display method according to claim 11, comprising:
an edge enhancing step for enhancing edges of the morphological information image read in the second reading step and outputting an edge-enhanced image;
wherein the superimposition displaying step displays in superimposition the measurement information image read in the first reading step and the edge-enhanced image with the edges enhanced in the edge enhancing step.

13. The display method according to claim 11 or 12, comprising:
a reduced displaying step for displaying in reduction at least one of the measurement information image read in the first reading step and the morphological information image read in the second reading step;
an image selecting step for selecting the image displayed in reduction in the reduced displaying step; and
an enlarged displaying step for displaying in enlargement the image selected in the image selecting step.

14. The display method according to claim 13, wherein:
the reduced displaying step displays in reduction an image after a superimposition process which superimposes the measurement information image and the morphological information image;
the image selecting step selects also the image after the superimposition process displayed in reduction in the reduced displaying step; and
the enlarged displaying step, when the image after the superimposition process is selected in the image selecting step, displays the image after the superimposition process in enlargement.

15. A display program for causing a computer to execute a series of processes for displaying measurement data obtained by measuring light generated from a luminescent or fluorescent substance included in a liquid to be measured, or radiation included in the liquid to be measured, the display program comprising:
a first reading step for reading a measurement information image having measurement information on the light generated from the luminescent or fluorescent substance included in the liquid to be measured, or the radiation included in the liquid to be measured;
a second reading step for reading a morphological information image having morphological information on the liquid to be measured; and
a superimposition displaying step for displaying in superimposition the measurement information image read in the first reading step and the morphological information image read in the second reading step;
the computer being caused to carry out processes in the above steps.
